# EUROPEAN PATENT APPLICATION

(11) **EP 4 663 192 A1**
(43) Date of publication of application: **17.12.2025**
(21) Application number: 24752891.2
(22) Date of filing: 07.02.2024
(51) Int. Cl.: A61K 31/519, A61K 47/20, A61K 47/12, A61K 47/14, A61K 9/00, A61P 37/00, A61P 17/00

(54) **PHARMACEUTICAL COMPOSITION FOR LOCAL ADMINISTRATION, PREPARATION METHOD THEREFOR, AND USE THEREOF**

(30) Priority: 07.02.2023 CN 202310097491
(71) Applicant: Reistone Biopharma Company Limited, Shanghai 201210 (CN)
(72) Inventor: SUN, Longwei, Shanghai 201210 (CN); HOU, Yanting, Shanghai 201210 (CN); YANG, Guang, Shanghai 201210 (CN); ZHU, Jiajun, Shanghai 201210 (CN)
(74) Representative: dompatent
(86) International application number: PCT/CN2024/076571
(87) International publication number: WO 2024/165036

(57) **Abstract**

The present disclosure relates to a pharmaceutical composition for local administration, a preparation method therefor, and the use thereof, and particularly relates to a pharmaceutical composition, comprising the following components: an active ingredient, dimethyl sulfoxide and a penetration enhancer. The active ingredient is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrol-2(1H)-carboxamide or pharmaceutically acceptable salts thereof. The penetration enhancer is one or more selected from lactic acid, isopropyl myristate and propylene glycol monocaprylate. The pharmaceutical composition has good skin penetration capability, is stable and is easy to apply.

## Description

The present application claims priority to Chinese Patent Application No. 202310097491.5 filed on Feb. 07, 2023, which is incorporated herein by reference in its entirety.

### TECHNICAL FIELD

The present disclosure belongs to the field of pharmaceutical formulations, and particularly relates to a pharmaceutical composition of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof and dimethyl sulfoxide.

### BACKGROUND

Atopic dermatitis (AD) is a chronic inflammatory skin disease, which is characterized by dry scaly skin, erythema, lesions with oozing and crusting, excoriations due to itch, and lichenification. Skin disorders are accompanied by intense pruritus, which poses a significant burden on the subject's quality of life. About 3% of adults and between 15% and 25% of children in the population have atopic dermatitis.

JAK (Janus kinase, including JAK1, JAK2, JAK3, and TYK2) is a family of intracellular, non-receptor tyrosine kinases. The downstream substrates of JAK include signal transducer and activator of transcription (STAT) proteins. STAT proteins function both as signaling molecules and transcription factors, and ultimately bind to specific DNA sequences present in the promoters of cytokine-responsive genes. JAK-STAT signaling is involved in the mediation of many abnormal immune responses such as allergies, asthma, autoimmune diseases such as transplant rejection, rheumatoid arthritis, amyotrophic lateral sclerosis, atopic dermatitis, alopecia areata, vitiligo, as well as various hematological and solid tumors.

Spleen tyrosine kinase (SYK) and JANUS kinase (JAK) are tyrosine kinases that play important roles in the pathogenesis of various types of autoimmune and inflammatory diseases, including atopic dermatitis, alopecia areata, and the like. Therefore, inhibition of SYK and JANUS kinase activities provides a potential approach for treating various types of inflammatory disorders.

Given the usefulness of JAK inhibitors in treating skin disorders, there is a need for improved topical formulations of JAK inhibitors. However, (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide and pharmaceutically acceptable salts thereof have a poor ability to penetrate the skin barrier. In particular, there is a need for stable and easily administrable formulations of targeted JAK inhibitors with good skin penetration properties.

### SUMMARY

The technical problem to be solved by the present disclosure is that the existing JAK inhibitors have a poor ability to penetrate the skin barrier and poor solubility, and thus, the present disclosure provides a pharmaceutical composition for topical administration, a preparation method therefor, and use thereof. The pharmaceutical composition comprises a JAK inhibitor with good skin penetration properties and is stable and easy to administer.

The present disclosure provides a pharmaceutical composition comprising the following components: an active ingredient, dimethyl sulfoxide, and a penetration enhancer, wherein the active ingredient is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof; the penetration enhancer is one or more penetration enhancers selected from lactic acid, isopropyl myristate, and propylene glycol monocaprylate.

In some embodiments, based on the total mass of the pharmaceutical composition, the content of dimethyl sulfoxide is 20%-80%, e.g., 20%, 25%, 30%, 35%, 40%, 45%, 50%, 55%, 60%, 65%, 70%, 75%, 80%, or any value therebetween.

In some embodiments, based on the total mass of the pharmaceutical composition, the content of the active ingredient is 0.3%-2.0%, e.g., 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, or any value therebetween.

In some embodiments, based on the total mass of the pharmaceutical composition, the content of water in the pharmaceutical composition is less than 10%.

In some embodiments, the pharmaceutical composition comprises the following components: an active ingredient, dimethyl sulfoxide, and a penetration enhancer, wherein the active ingredient is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof; the penetration enhancer is one or more penetration enhancers selected from lactic acid, isopropyl myristate, and propylene glycol monocaprylate; based on the total mass of the pharmaceutical composition, the content of dimethyl sulfoxide is 20%-80%, the content of the active ingredient is 0.3%-2.0%, and the content of water in the pharmaceutical composition is less than 10%.

In some embodiments, based on the total mass of the pharmaceutical composition, the content of the active ingredient may be 0.5%-2.0%, e.g., 0.5%, 1%, 1.5%, 1.855%, or 2%. In some embodiments, based on the total mass of the pharmaceutical composition, the content of the active ingredient may be 1%-2%. In some embodiments, based on the total mass of the pharmaceutical composition, the content of the active ingredient may be 0.5%-1%. In some embodiments, the active ingredient is present in a dissolved form in the pharmaceutical composition.

In some embodiments, based on the total mass of the pharmaceutical composition, the content of dimethyl sulfoxide may be 25%-55%. In some embodiments, based on the total mass of the pharmaceutical composition, the content of dimethyl sulfoxide may be 30%-45%, e.g., 35%, 40%, or 45%.

In some embodiments, based on the total mass of the pharmaceutical composition, the content of the penetration enhancer may be 1%-10%. In some embodiments, based on the total mass of the pharmaceutical composition, the content of the penetration enhancer may be 5%-10%, e.g., 5.5%, 7%, or 7.5%.

In some embodiments, based on the total mass of the pharmaceutical composition, the content of water may be less than 9%. In some embodiments, based on the total mass of the pharmaceutical composition, the content of water may be less than 8%. In some embodiments, based on the total mass of the pharmaceutical composition, the content of water may be less than 7%, e.g., less than 6%, less than 5%, less than 4%, less than 3%, less than 2%, or less than 1%.

In some embodiments, the pharmaceutical composition further comprises an organic solvent component other than dimethyl sulfoxide, and the organic solvent is known to those skilled in the art. In some embodiments, based on the total mass of the pharmaceutical composition, the content of the organic solvent component may be 10%-80% (e.g., 10%, 15%, 20%, 25%, 30%, 35%, 40%, 45%, 50%, 55%, 60%, 65%, 70%, 75%, 80%, or any value therebetween). In some embodiments, based on the total mass of the pharmaceutical composition, the content of the organic solvent component may be 10%-60%, e.g., 12%, 12.145%, 14%, 21%, 44%, 49.4%, 52%, 52.4%, 52.5%, 53.5%, or 54%. In some embodiments, based on the total mass of the pharmaceutical composition, the content of the organic solvent component may be 10%-40%. In some embodiments, based on the total mass of the pharmaceutical composition, the content of the organic solvent component may be 25%-40%. In some embodiments, the organic solvent component is one or more organic solvent components independently selected from benzyl alcohol, oleic acid, oleyl alcohol, diethylene glycol monoethyl ether, ethanol, and propylene glycol. In some embodiments, the organic solvent component is one or more organic solvent components selected from benzyl alcohol, diethylene glycol monoethyl ether, ethanol, and propylene glycol. In some embodiments, the organic solvent component is selected from diethylene glycol monoethyl ether and/or propylene glycol.

In some embodiments, the pharmaceutical composition further comprises a matrix, and the matrix may be a water-soluble matrix. In some embodiments, the water-soluble matrix is a polyethylene glycol polymer compound. In some embodiments, the polyethylene glycol polymer compound may have an average molecular weight of 100-6000. In optional embodiments, the polyethylene glycol polymer compound may have an average molecular weight of 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, or 6000. In some embodiments, the water-soluble matrix is polyethylene glycol 400 and polyethylene glycol 4000. In some embodiments, the water-soluble matrix is polyethylene glycol 400.

In some embodiments, the matrix is polyethylene glycol 400 and polyethylene glycol 4000, and the mass ratio of polyethylene glycol 400 to polyethylene glycol 4000 is (0.1-10):1. In some embodiments, the mass ratio of polyethylene glycol 400 to polyethylene glycol 4000 is (0.1-5):1 (e.g., 0.1:1, 0.3:1, or 9:35 (about 0.26:1)), so as to adjust the ointment to a suitable viscosity and spreadability and to ensure the content uniformity of the active ingredient.

In some embodiments, based on the total mass of the pharmaceutical composition, the content of the matrix may be 30%-80% (e.g., 30%, 35%, 40%, 45%, 50%, 55%, 60%, 65%, 70%, 75%, 80%, or any value therebetween). In some embodiments, based on the total mass of the pharmaceutical composition, the content of the matrix may be 40%-50%, e.g., 44% or 45.5%. In some embodiments, based on the total mass of the pharmaceutical composition, the content of the matrix is 1%-20% (e.g., 1%, 2%, 3%, 4%, 5%, 6%, 7%, 8%, 9%, 10%, 11%, 12%, 13%, 14%, 15%, 16%, 17%, 18%, 19%, 20%, or any value therebetween). In some embodiments, based on the total mass of the pharmaceutical composition, the content of the matrix is 5%-15%.

In some embodiments, the pharmaceutical composition comprises an antioxidant, which is known to those skilled in the art. In some embodiments, the antioxidant is one or more of butylated hydroxyanisole, butylated hydroxytoluene, propyl gallate, and tocopherol. In some embodiments, the antioxidant is butylated hydroxytoluene. In some embodiments, based on the total mass of the pharmaceutical composition, the content of the antioxidant may be 0.05%-0.5% (e.g., 0.05%, 0.1%, 0.15%, 0.2%, 0.25%, 0.3%, 0.35%, 0.4%, 0.45%, 0.5%, or any value therebetween). In some embodiments, based on the total mass of the pharmaceutical composition, the content of the antioxidant may be 0.05%-0.2%, e.g., 0.05% or 0.1%.

In some embodiments, the pharmaceutical composition comprises a moisturizer, which is known to those skilled in the art. In some embodiments, the moisturizer is one or more of glycerol, propylene glycol, 1,3-butanediol, sorbitol, xylitol, hyaluronic acid, and trehalose. In some embodiments, the moisturizer is glycerol. In some embodiments, based on the total mass of the pharmaceutical composition, the content of the moisturizer may be 1%-50% (e.g., 1%, 5%, 10%, 15%, 20%, 25%, 30%, 35%, 40%, 45%, 50%, or any value therebetween). In some embodiments, based on the total mass of the pharmaceutical composition, the content of the moisturizer may be 1%-25%, e.g., 5% or 20%. In some embodiments, based on the total mass of the pharmaceutical composition, the content of the moisturizer may be 10%-20%. In some embodiments, based on the total mass of the pharmaceutical composition, the content of the moisturizer may be 12%-18%.

In some embodiments, the pharmaceutical composition comprises a preservative, which is known to those skilled in the art. In some embodiments, the preservative is one or more of methylparaben, ethylparaben, benzoic acid, sorbic acid, phenoxyethanol, chlorobutanol, phenylmercuric acetate, phenol, cresol, and benzalkonium chloride. In some embodiments, the preservative is ethylparaben. In some embodiments, based on the total mass of the pharmaceutical composition, the content of the preservative is 0.05%-0.5% (e.g., 0.05%, 0.1%, 0.15%, 0.2%, 0.25%, 0.3%, 0.35%, 0.4%, 0.45%, 0.5%, or any value therebetween), e.g., 0.2%. In some embodiments, the pharmaceutical composition comprises a thickener, which is known to those skilled in the art. In some embodiments, the thickener is one or more of xanthan gum, hydroxyethyl cellulose, hydroxypropyl cellulose, methyl cellulose, sodium alginate, and poloxamer. In some embodiments, the thickener is hydroxypropyl cellulose. In some embodiments, based on the total mass of the pharmaceutical composition, the content of the thickener may be 0.1%-5% (e.g., 0.1%, 0.5%, 1%, 1.5%, 2%, 2.5%, 3%, 3.5%, 4%, 4.5%, 5%, or any value therebetween). In some embodiments, based on the total mass of the pharmaceutical composition, the content of the thickener may be 0.5%-3%, e.g., 1% or 2%. In some embodiments, based on the total mass of the pharmaceutical composition, the content of the thickener may be 2%-3%. In some embodiments, based on the total mass of the pharmaceutical composition, the content of the thickener may be 0.1%-15% (e.g., 0.1%, 1%, 2%, 3%, 4%, 5%, 6%, 7%, 8%, 9%, 10%, 11%, 12%, 13%, 14%, 15%, or any value therebetween). In some embodiments, based on the total mass of the pharmaceutical composition, the content of the thickener may be 5%-15%.

In some embodiments, the pharmaceutical composition comprises a thickener, which is one or more thickeners selected from carbomer and siloxane substances. In some embodiments, the siloxane substance is one or more siloxane substances selected from cyclic dimethylsiloxane, poly(dimethylsiloxane), and ST-Elastomer 10.

In some embodiments, the pharmaceutical composition has a pH of < 7. In some embodiments, the pharmaceutical composition has a pH selected from 3-6.5 (e.g., 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, or any value therebetween). In some embodiments, the pharmaceutical composition has a pH selected from 3.5-6.0. In some embodiments, the pharmaceutical composition has a pH selected from 3.5-4.5. In some embodiments, the reagent for adjusting the pH is one or more reagents selected from hydrochloric acid, sodium hydroxide, and triethanolamine.

In some embodiments, the pharmaceutical composition comprises a taste-masking agent, which is known to those skilled in the art. In some embodiments, the taste-masking agent is one or more of strawberry essence, orange essence, and cyclodextrin.

In some embodiments, the pharmaceutical composition comprises an active ingredient, dimethyl sulfoxide, an organic solvent, a thickener, an antioxidant, and a penetration enhancer, wherein the organic solvent is one or more of benzyl alcohol, oleic acid, oleyl alcohol, diethylene glycol monoethyl ether, ethanol, and propylene glycol; the thickener is one or more of xanthan gum, hydroxyethyl cellulose, hydroxypropyl cellulose, methyl cellulose, and poloxamer; the antioxidant is one or more of butylated hydroxyanisole, butylated hydroxytoluene, propyl gallate, and tocopherol; the penetration enhancer is one or more penetration enhancers selected from lactic acid, isopropyl myristate, and propylene glycol monocaprylate, wherein the content of each component is as described in any one of the previous embodiments of the present disclosure. In optional embodiments, the pharmaceutical composition comprises:
1) 0.3%-2.0% (e.g., 0.5%-2.0%, or e.g., 1%-2%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide,
3) 10%-80% (e.g., 10%-60%) by mass of an organic solvent,
4) 0.1%-5% (e.g., 0.5%-3%) by mass of a thickener,
5) 0.05%-0.5% (e.g., 0.05%-0.2%) by mass of an antioxidant, and
6) 1%-10% (e.g., 5%-10%) by mass of a penetration enhancer.

In optional embodiments, the pharmaceutical composition comprises components in any one of the following groups:
1) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 40% by mass of dimethyl sulfoxide, 49.1% by mass of diethylene glycol monoethyl ether, 0.3% by mass of benzyl alcohol, 2% by mass of hydroxypropyl cellulose, 0.1% by mass of butylated hydroxytoluene, and 7.5% by mass of isopropyl myristate;
   or
2) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 40% by mass of dimethyl sulfoxide, 49.1% by mass of diethylene glycol monoethyl ether, 3.3% by mass of oleic acid, 1% by mass of hydroxypropyl cellulose, 0.1% by mass of butylated hydroxytoluene, and 5.5% by mass of isopropyl myristate.

In some embodiments, the pharmaceutical composition comprises an active ingredient, dimethyl sulfoxide, an organic solvent, a water-soluble matrix, and a penetration enhancer, wherein the organic solvent is one or more of benzyl alcohol, oleic acid, oleyl alcohol, diethylene glycol monoethyl ether, ethanol, and propylene glycol; the water-soluble matrix is a polyethylene glycol polymer compound; the penetration enhancer is one or more penetration enhancers selected from lactic acid, isopropyl myristate, and propylene glycol monocaprylate, wherein the content of each component is as described in any one of the previous embodiments of the present disclosure.

In optional embodiments, the pharmaceutical composition comprises:
1) 0.3%-2.0% (e.g., 0.5%-2.0%, or e.g., 1%-2%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide,
3) 10%-80% (e.g., 10%-60%) by mass of an organic solvent,
4) 30%-80% (e.g., 40%-50%) by mass of a water-soluble matrix, and
5) 1%-10% (e.g., 5%-10%) by mass of a penetration enhancer.

In optional embodiments, the pharmaceutical composition comprises components in any one of the following groups:
1) 0.5% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 5% by mass of diethylene glycol monoethyl ether, 5% by mass of propylene glycol, 2% by mass of benzyl alcohol, 7% by mass of lactic acid, 10.5% by mass of polyethylene glycol 400, and 35% by mass of polyethylene glycol 4000;
2) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 4% by mass of propylene glycol, 7% by mass of lactic acid, 4% by mass of polyethylene glycol 400, and 40% by mass of polyethylene glycol 4000;
3) 1.5% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 4% by mass of propylene glycol, 7% by mass of lactic acid, 9% by mass of polyethylene glycol 400, and 35% by mass of polyethylene glycol 4000;
4) 1.855% by mass of bisulfate of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 2.145% by mass of propylene glycol, 7% by mass of lactic acid, 9% by mass of polyethylene glycol 400, and 35% by mass of polyethylene glycol 4000;
   or
5) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 4% by mass of propylene glycol, 7% by mass of lactic acid, 9% by mass of polyethylene glycol 400, and 35% by mass of polyethylene glycol 4000.

In some embodiments, the pharmaceutical composition comprises an active ingredient, dimethyl sulfoxide, and a penetration enhancer, wherein the penetration enhancer is one or more penetration enhancers selected from lactic acid, isopropyl myristate, and propylene glycol monocaprylate, and the pharmaceutical composition comprises:
1) 0.3%-2.0% (e.g., 0.5-2.0%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide, and
3) 1%-10% (e.g., 5%-10%) by mass of a penetration enhancer.

In some embodiments, the pharmaceutical composition comprises an active ingredient, dimethyl sulfoxide, a penetration enhancer, and an organic solvent, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol. In optional embodiments, the pharmaceutical composition comprises:
1) 0.3%-2.0% (e.g., 0.5-2.0%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide,
3) 1%-10% (e.g., 5%-10%) by mass of a penetration enhancer, and
4) 10%-80% (e.g., 10%-60%) by mass of an organic solvent.

In optional embodiments, the pharmaceutical composition comprises the following components:
1% by mass of bisulfate of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 12.5% by mass of propylene glycol, 10% by mass of ethanol, and 7% by mass of lactic acid.

In some embodiments, the pharmaceutical composition comprises an active ingredient, dimethyl sulfoxide, a penetration enhancer, an organic solvent, a thickener, and a water-soluble matrix, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol; the thickener is one or more of xanthan gum, hydroxyethyl cellulose, hydroxypropyl cellulose, methyl cellulose, and poloxamer; the water-soluble matrix is a polyethylene glycol polymer compound. In optional embodiments, the pharmaceutical composition comprises:
1) 0.3%-2.0% (e.g., 0.5-2.0%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide,
3) 1%-10% (e.g., 5%-10%) by mass of a penetration enhancer,
4) 10%-80% (e.g., 10%-60%) by mass of an organic solvent,
5) 0.1%-15% (e.g., 1%-15%) by mass of a thickener, and
6) 1%-20% (e.g., 5%-15%) or 30%-80% (e.g., 40%-50%) by mass of a water-soluble matrix.

In some embodiments, the pharmaceutical composition comprises an active ingredient, dimethyl sulfoxide, a penetration enhancer, an organic solvent, a thickener, a water-soluble matrix, and an antioxidant, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol; the thickener is one or more thickeners selected from carbomer and siloxane substances; the water-soluble matrix is a polyethylene glycol polymer compound; the antioxidant is one or more of butylated hydroxyanisole, butylated hydroxytoluene, propyl gallate, and tocopherol. In optional embodiments, the pharmaceutical composition comprises:
1) 0.3%-2.0% (e.g., 0.5-2.0%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide,
3) 1%-10% (e.g., 5%-10%) by mass of a penetration enhancer,
4) 10%-80% (e.g., 10%-60%) by mass of an organic solvent,
5) 0.1%-15% (e.g., 1%-15%) by mass of a thickener,
6) 1%-20% (e.g., 5%-15%) by mass of a water-soluble matrix, and
7) 0.05%-0.5% (e.g., 0.05%-0.2%) by mass of an antioxidant.

In optional embodiments, the pharmaceutical composition comprises the following components:
0.37% by mass of bisulfate of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 0.05% by mass of butylated hydroxytoluene, 2% by mass of benzyl alcohol, 9.63% by mass of propylene glycol, 7% by mass of lactic acid, 12.5% by mass of polyethylene glycol 400, 2.3% by mass of carbomer, 7% by mass of cyclic dimethylsiloxane, 1% by mass of poly(dimethylsiloxane), and 2% by mass of ST-Elastomer 10.

In some embodiments, the pharmaceutical composition comprises an active ingredient, dimethyl sulfoxide, an organic solvent, a penetration enhancer, a water-soluble matrix, a thickener, a moisturizer, and an antioxidant, wherein the organic solvent is one or more of benzyl alcohol, oleic acid, oleyl alcohol, diethylene glycol monoethyl ether, ethanol, and propylene glycol; the penetration enhancer is one or more penetration enhancers selected from lactic acid, isopropyl myristate, and propylene glycol monocaprylate; the water-soluble matrix is a polyethylene glycol polymer compound; the thickener is one or more of xanthan gum, hydroxyethyl cellulose, hydroxypropyl cellulose, methyl cellulose, sodium alginate, and poloxamer; the moisturizer is one or more of glycerol, propylene glycol, 1,3-butanediol, sorbitol, xylitol, hyaluronic acid, and trehalose; the antioxidant is one or more of butylated hydroxyanisole, butylated hydroxytoluene, propyl gallate, and tocopherol. In optional embodiments, the pharmaceutical composition comprises:
1) 0.3%-2.0% (e.g., 0.5%-2.0%) by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof,
2) 20%-80% (e.g., 25%-55%, or e.g., 30%-45%) by mass of dimethyl sulfoxide,
3) 10%-80% (e.g., 10%-60%) by mass of an organic solvent,
4) 1%-10% (e.g., 5%-10%) by mass of a penetration enhancer,
5) 1%-20% (e.g., 5%-15%) by mass of a water-soluble matrix,
6) 0.1%-5% (e.g., 0.5%-3%) by mass of a thickener,
7) 0.05%-0.5% (e.g., 0.05%-0.2%) by mass of an antioxidant, and
8) 1%-50% (e.g., 10%-20%) by mass of a moisturizer.

In optional embodiments, the pharmaceutical composition comprises components in any one of the following groups:
(1) 0.5% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 0.2% by mass of butylated hydroxytoluene, 10% by mass of diethylene glycol monoethyl ether, 12%-18% by mass of glycerol, 7% by mass of lactic acid, 9% by mass of polyethylene glycol 400, 15%-23% by mass of propylene glycol, and 2%-3% by mass of hydroxypropyl cellulose;
(2) 1.0% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 0.2% by mass of butylated hydroxytoluene, 10% by mass of diethylene glycol monoethyl ether, 12%-18% by mass of glycerol, 7% by mass of lactic acid, 9% by mass of polyethylene glycol 400, 15%-23% by mass of propylene glycol, 2%-3% by mass of hydroxypropyl cellulose.

In some embodiments, the pharmaceutical composition is a pharmaceutical composition for topical use. In some embodiments, the pharmaceutical composition is selected from a gel, an emulsion, or an ointment. In some embodiments, the pharmaceutical composition is selected from a gel.

The present disclosure also provides a method for preparing the aforementioned pharmaceutical composition, comprising: mixing the components. In some embodiments, the active ingredient, dimethyl sulfoxide, and the penetration enhancer are mixed, and then, the resulting mixture is mixed with optionally at least one of the organic solvent, the matrix, and the antioxidant.

In some embodiments, the method comprises: mixing the active ingredient and dimethyl sulfoxide, and further mixing with optionally at least one of the penetration enhancer, the organic solvent, the matrix, the antioxidant, the moisturizer, the thickener, and the preservative.

In some embodiments, the active ingredient is mixed with dimethyl sulfoxide and the antioxidant to obtain a mixture I. In some embodiments, the mixture I is mixed with at least one of the penetration enhancer, the organic solvent, the matrix, the moisturizer, and the preservative to obtain a mixture II. In some embodiments, the mixture II is mixed with the thickener.

In some embodiments, the method further comprises a pH adjustment step of adjusting the pH to < 7 (e.g., 3.0-6.5, 3.5-6.0, or 3.5-4.5). In some embodiments, the pH of the mixture II is adjusted to < 7 (e.g., 3.0-6.5, 3.5-6.0, or 3.5-4.5).

In some embodiments, the active ingredient is mixed with dimethyl sulfoxide and the antioxidant to obtain a mixture I, the mixture I is then mixed with at least one of the penetration enhancer, the organic solvent, the matrix, the moisturizer, and the preservative to obtain a mixture II, the pH is adjusted to < 7 (e.g., 3.0-6.5, 3.5-6.0, or 3.5-4.5), and the mixture II is further mixed with the thickener.

In some embodiments, the method comprises: mixing the active substance with dimethyl sulfoxide, and further mixing with optionally at least one of the organic solvent, the matrix, the antioxidant, the moisturizer, the thickener, and the preservative. In some embodiments, the thickener is mixed with the organic solvent in advance.

The present disclosure also provides use of the aforementioned pharmaceutical composition in the manufacture of a medicament for treating or preventing immune system disorders or diseases.

In some embodiments, the immune system disorders or diseases may be selected from diseases of the immune system, comprising, for example, organ transplant rejection (e.g., allograft rejection and graft versus host disease); autoimmune diseases, comprising, for example, lupus, multiple sclerosis, rheumatoid arthritis, juvenile arthritis, psoriasis, ulcerative colitis, Crohn's disease, autoimmune thyroid diseases, and the like; skin diseases, comprising, for example, psoriasis, rash, atopic dermatitis, and the like; allergic disorders, comprising, for example, asthma, rhinitis, and the like; viral diseases, comprising, for example, hepatitis B, hepatitis C, chickenpox, herpes zoster virus, and the like; type I diabetes and diabetic complications; Alzheimer's disease, xerophthalmia, myelofibrosis, thrombocythemia, polycythemia, or leukemia; cancers, comprising, for example, solid tumors (e.g., prostate cancer, kidney cancer, pancreatic cancer, gastric cancer, breast cancer, lung cancer, head and neck cancer, thyroid cancer, glioblastoma, melanoma, and the like), hematological cancers (e.g., lymphoma, leukemia, and the like), skin cancers (e.g., cutaneous T-cell lymphoma, cutaneous B-cell lymphoma), and the like.

In optional embodiments, provided is use of the aforementioned pharmaceutical composition in the manufacture of a medicament for treating skin diseases.

In some embodiments, the immune system disorders or diseases are selected from organ transplant rejection (e.g., allograft rejection and graft versus host disease), ankylosing spondylitis, active non-radiographic axial spondyloarthritis, rheumatoid arthritis, psoriatic arthritis, ulcerative colitis, psoriasis, vitiligo, alopecia areata, atopic dermatitis, Crohn's disease, and lymphoma. In some embodiments, the immune system disorders or diseases are selected from vitiligo.

The present disclosure also provides use of the aforementioned pharmaceutical composition in the manufacture of a medicament for preventing and/or treating diseases, wherein the diseases are selected from organ transplant rejection (e.g., allograft rejection and graft versus host disease), ankylosing spondylitis, active non-radiographic axial spondyloarthritis, rheumatoid arthritis, psoriatic arthritis, ulcerative colitis, psoriasis, vitiligo, alopecia areata, atopic dermatitis, Crohn's disease, and lymphoma. In some embodiments, the diseases are selected from vitiligo.

The pharmaceutically acceptable salt of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide described in the present disclosure is one or more of hydrochloride, maleate, hydrobromide, p-toluenesulfonate, mesylate, sulfate, bisulfate, and ethanesulfonate. In some embodiments, the pharmaceutically acceptable salt is bisulfate.

In the pharmaceutical compositions of the present disclosure, no additional water is added except for a trace amount of water that may be contained in the components.

The term "penetration enhancer" in the present disclosure, in addition to the aforementioned one or more of lactic acid, isopropyl myristate, and propylene glycol monocaprylate, may be further selected from other excipients capable of promoting the transdermal absorption of the active ingredient,.

On the basis of not violating common senses in the art, the above various preferred conditions can be combined arbitrarily to obtain various preferred embodiments of the present disclosure.

The reagents and raw materials used in the present disclosure are commercially available.

The positive and progressive effects of the present disclosure are as follows: provided is a pharmaceutical composition, which comprises a JAK inhibitor with good skin penetration properties and is stable and easy to administer.

### BRIEF DESCRIPTION OF THE DRAWINGS

FIG. 1 shows a PLM image of the blank dissolved-type ointment containing PEG4000 + DMSO of Example 5.7.
FIG. 2 shows a PLM image of the API.
FIG. 3 shows a PLM image of the dissolved-type ointment containing 1% API + PEG4000 + DMSO + LA of Example 5.5.
FIG. 4 shows a PLM image of the ointment containing 1% API + PEG4000 + DMSO of Example 5.6.
FIG. 5 shows a PLM image of the 1% suspension-type ointment of Example 7.2.

### DETAILED DESCRIPTION

The present disclosure is further illustrated by way of examples below, but is not therefore limited to the scope of those examples. The experimental methods in the examples below of which the specific conditions are not indicated are selected according to the conventional methods and conditions, or according to the commodity instructions.

### Example 1. Synthesis of Active Ingredient

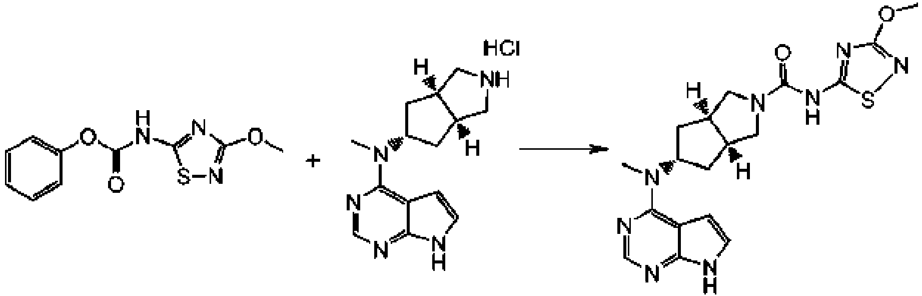

Prepared according to Example 1 in the patent CN108884100B.

### Example 2. Preparation of Bisulfate

Prepared according to the examples in the patent CN105980390B.

### Example 3. Preparation of Solutions

According to the formulations in Table 1, a certain amount of the active ingredient was dissolved in dimethyl sulfoxide of the formulation until it became a clear solution, and then the formulation amount of an additional non-aqueous solvent benzyl alcohol, oleyl alcohol, or oleic acid was added. Hydroxypropyl cellulose was uniformly swelled in diethylene glycol monoethyl ether, and the dimethyl sulfoxide solvent and the diethylene glycol monoethyl ether phase were mixed to obtain a clear and high-viscosity solution.

As shown in Table 2, under the condition that the concentration of the active ingredient was 1%, dimethyl sulfoxide was replaced with other organic solvents such as ethanol, propylene glycol, polyethylene glycol 400, or dimethylformamide, and the solutions were all suspensions. Meanwhile, as shown in Table 3, even if the concentration of dimethyl sulfoxide was maintained at a high level, the addition of a trace amount of water failed to prepare a clear solution above 1% concentration.

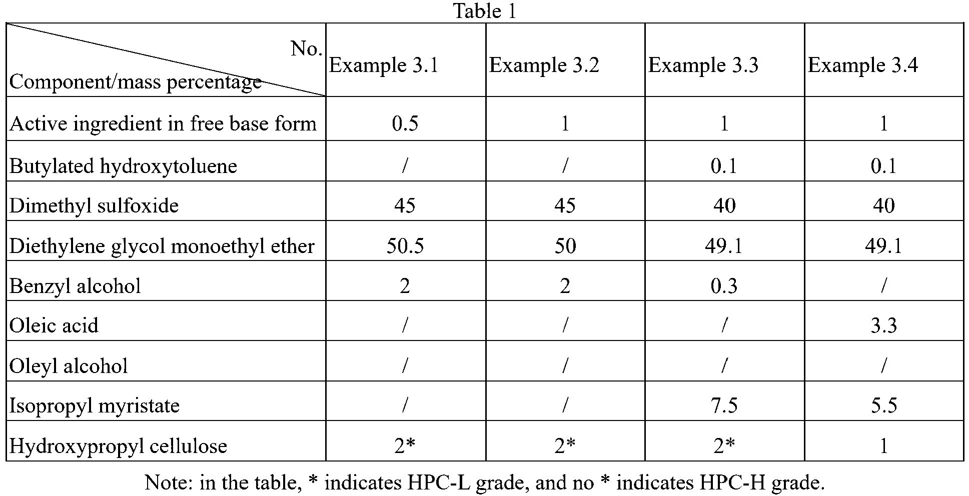

**Table 2**

| Component | Mass percentage | | | | | | | |
|---|---|---|---|---|---|---|---|---|
| Active ingredient in free base form | 1.5 | 1 | 1 | 1 | 1 | 1 | 1 | 1 |
| Dimethyl sulfoxide | 45 | | | | | | | 10 |
| Diethylene glycol monoethyl ether | 40 | 40 | 40 | | | 40 | 40 | 40 |
| Dimethylformamide | | 45 | | 45 | | | | |
| Ethanol | | | | | 45 | | 50 | |
| Propylene glycol | 13.5 | 14 | 59 | 54 | 54 | | 9 | |
| Polyethylene glycol 400 | | | | | | 59 | | 49 |
| Phenomenon | Clear | Turbid | Turbid | Turbid | Turbid | Turbid | Turbid | Turbid |

**Table 3**

| Component | Mass percentage | | | | | | |
|---|---|---|---|---|---|---|---|
| Active ingredient in free base form | 2 | 1 | 1 | 1 | 1 | 1 | 1 |
| Dimethyl sulfoxide | 45 | 40 | 35 | 35 | 40 | 40 | 40 |
| Diethylene glycol monoethyl ether | 40 | 40 | 30 | 10 | 35 | 15 | 15 |
| Glycerol | | 5 | 20 | 20 | | 20 | |
| Propylene glycol | 12 | 14 | 14 | 34 | 14 | 14 | 14 |
| Water | | | | | 10 | 10 | 30 |
| Phenomenon | Clear | Clear | Clear | Clear | Turbid | Turbid | Turbid |

### Example 4. Stability Results of Solutions

The solutions in Example 3 were placed under the conditions of 25 °C/60% RH and 40 °C/75% RH to test stability. The appearance was visually observed, and the content and related substances were detected by HPLC. The results are shown in Tables 4-5, and the anhydrous solutions had good physical and chemical stability.

### HPLC method for content detection:

| | | | |
|---|---|---|---|
| Chromatographic column | Waters SunFireRC18 5 µm 4.6*250 mm | | |
| Column temperature (°C) | 30 | Flow rate (mL/min) | 1.0 |
| Sample injection volume (µL) | 10 | Wavelength (nm) | 218 |
| Mobile phase | 0.01 mol/L diammonium hydrogen phosphate solution + 0.1% triethylamine (pH 6.2):acetonitrile = 60:40 | | |
| Diluent | Water:acetonitrile:1 mol/L NaOH = 60:40:1 | | |
| Sample concentration | 50 µg/mL | | |
| Elution mode | Isocratic elution | | |
| Run time | 10 min | | |

### HPLC method for related substances detection:

| | | | |
|---|---|---|---|
| Chromatographic column | Waters SunFireRC18 5 µm 4.6*250 mm | | |
| Column temperature (°C) | 30 | Flow rate (mL/min) | 1.0 |
| Sample injection volume (µL) | 10 | Wavelength (nm) | 218 |
| Mobile phase | Mobile phase A: 0.01 mol/L diammonium hydrogen phosphate solution + 0.1% triethylamine, pH 6.2 | | |
| | Mobile phase B: acetonitrile | | |
| Diluent | Water:acetonitrile:1 mol/L HCl = 80:20:1 | | |
| Sample concentration | 0.2 mg/mL | | |
| Standard concentration | 2 µg/mL | | |
| Elution mode | Gradient elution | | |
| Gradient program | Time (min) | Mobile phase A (%) | Mobile phase B (%) |
| | 0 | 90 | 10 |
| | 16 | 62 | 38 |
| | 35 | 20 | 80 |
| | 45 | 20 | 80 |
| | 45.1 | 90 | 10 |
| | 52 | 90 | 10 |

**Table 4**

| / | / | Time/month | Example 3.2 25 °C/60% RH condition | Example 3.3 25 °C/60% RH condition | Example 3.4 40 °C/75% RH condition |
|---|---|---|---|---|---|
| Appearance | Colorless or yellow, clear solution | 0 | Comply | Comply | Comply |
| | | 1 | Comply | Comply | Comply |
| | | 2 | Comply | Comply | Comply |
| | | 3 | Comply | Comply | Comply |
| | | 6 | Comply | Comply | Comply |
| Content (%) | | 0 | / | / | 100.7 |
| | | 1 | 99.2 | 100.5 | 100.4 |
| | | 6 | 96.3 | 97.4 | No detection |
| Related substances (total impurity%) | | 0 | / | / | 0.10 |
| | | 1 | 0.05 | 0.05 | 0.12 |
| | | 6 | 0.27 | 0.07 | 0.20 |

**Table 5. Stability data of Example 3.3**

| Sample | | State | Content (%) | Related substances (%) | |
|---|---|---|---|---|---|
| | | | | RRT: 0.92 min | RRT:0.93 min |
| Example 3.3 | Initial state | Clear solution | 98.48 | -- | -- |
| | 60 °C-5 days | Clear solution | -- | -- | -- |
| | 60 °C-10 days | Clear solution | 101.23 | -- | -- |
| | 60 °C-30 days | Clear solution | 100.73 | 0.08 | 0.05 |
| | 40 °C/75% RH-1 month | Yellowish solution | 100.94 | -- | 0.053 |

| | | | | | |
|---|---|---|---|---|---|
| Note: in the table, "RRT" indicates relative retention time, and "--" indicates undetectable. | | | | | |

### Example 5. Dissolved-Type Ointments

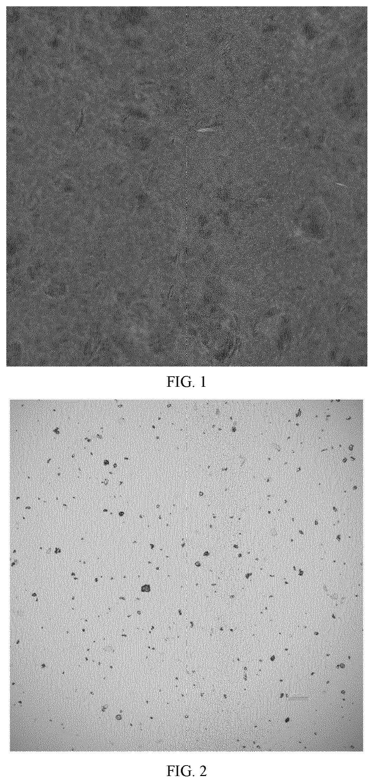

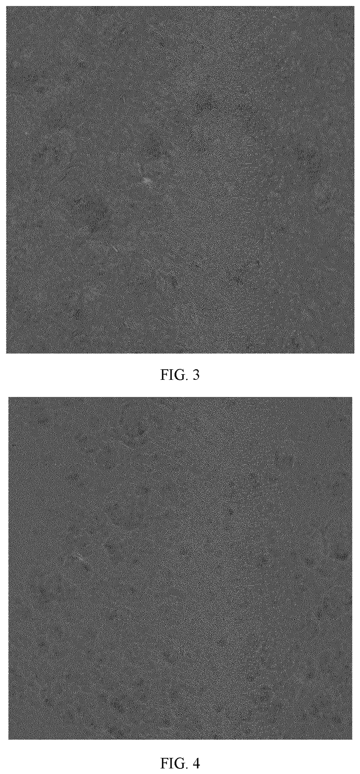

A certain amount of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide (free base) or a pharmaceutically acceptable salt thereof was dissolved in the formulation amount of dimethyl sulfoxide. The remaining solvents, diethylene glycol monoethyl ether, propylene glycol, benzyl alcohol, polyethylene glycol 400, lactic acid, and polyethylene glycol 4000, were added to the dimethyl sulfoxide solution and heated to 60 °C. The solution was clear and transparent, and was continuously stirred and cooled to room temperature. After solid was precipitated, the sample was homogenized at 4000 rpm for 10 min to finally form a semi-solid ointment formulation. As shown in PLM Figures 1-4, the formulations containing a high proportion of the DMSO solvent showed only polarization of the PEG matrix, and the API mainly existed in a dissolved form.

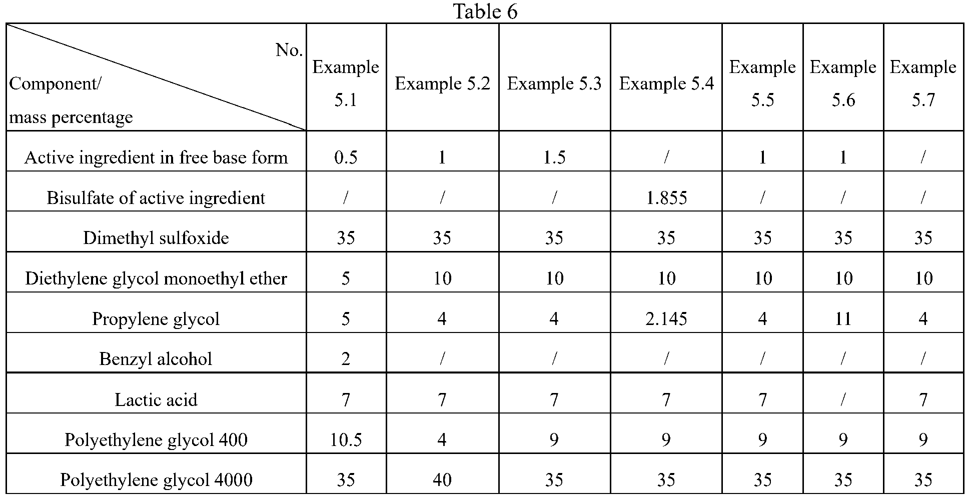

### Example 6. Stability Results of Ointments

The ointments in Example 5 were placed under the condition of 25 °C/60% RH to test stability. The appearance was visually observed, and the content and related substances were detected by HPLC. The dissolved-type ointments had good physical and chemical stability.

**Table 7**

| / | / | Time/month | Example 5.1 | Example 5.2 | Example 5.3 | Example 5.4 | Example 5.5 | Example 5.6 |
|---|---|---|---|---|---|---|---|---|
| Appearance | White or yellow ointment | 0 | Comply | Comply | Comply | Comply | Comply | Comply |
| | | 2 | Comply | Comply | Comply | Comply | Comply | Comply |
| Content (%) | | 0 | / | / | / | / | 94.9 | 94.4 |
| | | 2 | 93.8 | 94.1 | / | / | / | / |
| Related substances (total impurity%) | | 0 | / | / | / | / | 0.92 | 0.78 |
| | | 2 | 1.56 | 0.56 | / | / | / | / |

### Example 7. Preparation of Suspension-Type Ointments

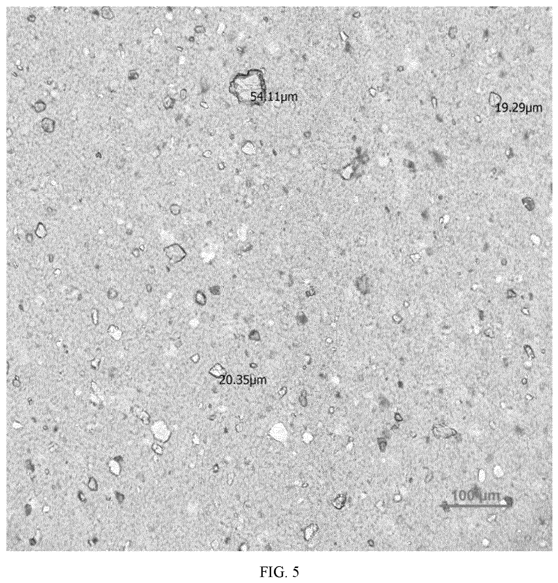

Certain amounts of micronized (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide (free base), diethylene glycol monoethyl ether, propylene glycol, glycerol, polyethylene glycol 400, butylated hydroxytoluene, and ethylparaben were heated to 70 °C, homogenized, stirred, and dispersed, and then polyethylene glycol 4000 was added until it melted. The mixture was homogenized and stirred continuously, and cooled to 40-50 °C, and as the ointment was stirred and homogenized continuously at this temperature, a white or off-white semi-solid ointment was gradually formed. The ointment was kept warm for later use. The PLM image of Example 7.2 is as shown in FIG. 5.

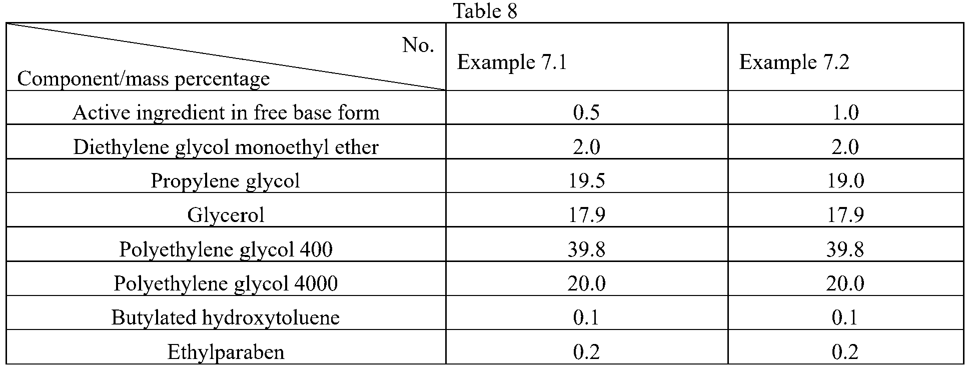

### Example 8

The penetrability of the API in the topical formulations was studied using freshly excised skin samples from Bama miniature pigs mounted in Franz diffusion cells. The fresh skin of Bama miniature pigs was depilated and then stored at -20 °C for later use. On the morning of the experiment, the skin was taken out and thawed, and after the transepidermal water loss value of the skin was detected to be less than 30, the skin was ready for use. The skin was cut to a size suitable for placing the pig skin samples between the donor and receiver compartments, and the pig skin samples were placed between the donor and receiver compartments of the Franz diffusion cell. The opening of the Franz cell was 1 cm², and about 200 mg of the sample was evenly applied to the pig skin. 8.0 mL of a receiver solution was used. At 0.5 h, 2 h, 4 h, 8 h, 12 h, and 24 h, 1.5 mL of the sample was removed and replaced with a new receiver solution. At 24 h, the skin was collected. The skin retention amount and drug penetration concentration were detected by HPLC.

### Experimental parameters of IVPT:

| Item | Parameter |
|---|---|
| Receiver solution | pH 7.0, 50 mM sodium citrate + 10% HP-β-CD |
| Receiver cell volume | 8.0 mL |
| Sample amount | 200 mg |
| Receiving area | 1.0 cm² |
| Temperature | 32.0±1 °C |
| Rotor speed | 250 rpm |
| Sampling volume | 1.5 mL |
| Supplementary liquid volume | 1.5 mL |
| Sampling time point | 0.5, 2, 4, 8, 12, 24 h |
| Residual drug and skin extract solution | Acetonitrile:water:1 M NaOH solution = 40:60:1 |

| Sample | 0.5% suspension-type ointment (see Example 7) | 1% suspension-type ointment (see Example 7) | 0.5% solution (see Example 3) | 1% solution (see Example 3) |
|---|---|---|---|---|
| Batch No. | Example 7.1 | Example 7.2 | Example 3.1 | Example 3.2 |
| Skin retention amount/µg | 8.0±5.8 | 6.5±4.1 | 21.0±6.6 | 61.2±20.7 |
| Drug penetration amount/µg | 0.139±0.121 | 0.007±0.008 | 5.3±0.5 | 8.3±3.0 |

| | | | | |
|---|---|---|---|---|
| Note: skin retention amount = epidermis drug amount + dermis drug amount. | | | | |

For solutions containing dimethyl sulfoxide, there was a general trend of increased skin retention amount and penetration amount when the concentration was increased from 0.5% to 1.0%, while this trend was not observed for the suspension-type ointments. Both the skin retention amount and penetration amount of the solutions were several times higher than the suspension-type ointments.

### Example 9. Emulsion

A certain amount of a pharmaceutically acceptable salt of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide was dissolved in dimethyl sulfoxide, butylated hydroxytoluene, propylene glycol, benzyl alcohol, lactic acid, polyethylene glycol 400, carbomer 980, and water, and the resulting mixture was heated to 50 °C and stirred until it was clear. Cyclic dimethylsiloxane, poly(dimethylsiloxane), ST-Elastomer 10, and carbomer were uniformly stirred and heated to 50 °C, and the resulting mixture was added to the drug-containing phase and stirred until it became a gel semisolid. After the pH value was adjusted to a specified value, the mixture was stirred continuously and naturally cooled to room temperature.

**Table 9**

| Component | Proportion (%) and Batch No. |
|---|---|
| | Example 9.1 |
| Bisulfate of active ingredient | 0.37 |
| Dimethyl sulfoxide | 35 |
| Butylated hydroxytoluene | 0.05 |
| Benzyl alcohol | 2 |
| Propylene glycol | 9.63 |
| Lactic acid | 7 |
| Polyethylene glycol 400 | 12.5 |
| Carbomer 980 | 1.5 |
| Water | 21.15 |
| Cyclic dimethylsiloxane | 7 |
| Poly(dimethylsiloxane) | 1 |
| ST-Elastomer 10 | 2 |
| Carbomer | 0.8 |
| pH (25% triethanolamine) | 5.5-5.9 |

### Example 10

The penetrability of the API in the topical formulations was studied using freshly excised skin samples from Bama miniature pigs mounted in Franz diffusion cells. The fresh skin of Bama miniature pigs was depilated and then stored at -20 °C for later use. On the morning of the experiment, the skin was taken out and thawed, and after the transepidermal water loss value of the skin was detected to be less than 30, the skin was ready for use. The skin was cut to a size suitable for placing the pig skin samples between the donor and receiver compartments, and the pig skin samples were placed between the donor and receiver compartments of the Franz diffusion cell. About 60 mg of the sample was evenly applied to the pig skin. The opening of the Franz cell was 3.14 cm². 8.0 mL of a receiver solution was used. At 2 h, 6 h, and 20 h, 1.0 mL of the sample was removed and replaced with a new receiver solution. At 20 h, the skin was collected. The pig skin was treated, the epidermal and dermis layers were separated and extracted with a skin extract solution, and drug concentrations in the epidermal and dermis layers were detected by HPLC.

### Experimental parameters of IVPT:

| Item | Parameter |
|---|---|
| Receiver solution | pH 7.0, 50 mM sodium citrate + 10% HP-β-CD |
| Receiver cell volume | 8.0 mL |
| Sample amount | 60 mg |
| Receiving area | 3.14 cm² |
| Temperature | 32.0±1 °C |
| Rotor speed | 250 rpm |
| Sampling volume | 1.0 mL |
| Supplementary liquid volume | 1.0 mL |
| Sampling time point | 2, 6, 20 h |
| Residual drug and skin extract solution | Acetonitrile:water:1 M NaOH solution = 40:60:1 |

| Sample | 0.5% suspension-type ointment (see Example 7) | 0.5% dissolved-type ointment (see Example 5) | 0.3% emulsion (see Example 9) |
|---|---|---|---|
| Batch No. | Example 7.1 | Example 5.1 | Example 9.1 |
| Epidermis drug amount/µg | 3.2±0.3 | 23.9±7.6 | 8.3±2.9 |
| Dermis drug amount/µg | 4.7±2.6 | 15.4±3.3 | 5.4±2.5 |
| Drug penetration amount/µg | Below limit of detection | Below limit of detection | Below limit of detection |

### Example 11

The active ingredient was dissolved in the formulation amount of dimethyl sulfoxide until it became a clear solution, and then the formulation amounts of other non-aqueous solvents propylene glycol, as well as a penetration enhancer were separately added to obtain a clear solution.

**Table 10**

| Component | Proportion (%) and Batch No. | | | |
|---|---|---|---|---|
| | Example 11.1 | Example 11.2 | Example 11.3 | Example 11.4 |
| Active ingredient in free base form | 1 | 1 | 1 | 1 |
| Dimethyl sulfoxide | 45 | 45 | 45 | 45 |
| Propylene glycol | 47 | 47 | 47 | 54 |
| Penetration Enhancer | Lactic acid 7 | Propylene glycol monocaprylate 7 | Polyoxyethylene 20 oleyl ether 7 | NA |

### Example 12

The penetrability of the API in the topical formulations was studied using freshly excised skin samples from Bama miniature pigs mounted in Franz diffusion cells. The fresh skin of Bama miniature pigs was depilated and then stored at -20 °C for later use. On the morning of the experiment, the skin was taken out and thawed, and after the transepidermal water loss value of the skin was detected to be less than 30, the skin was ready for use. The skin was cut to a size suitable for placing the pig skin samples between the donor and receiver compartments, and the pig skin samples were placed between the donor and receiver compartments of the Franz diffusion cell. About 60 mg of the sample was evenly applied to the pig skin. The opening of the Franz cell was 3.14 cm². 8.0 mL of a receiver solution was used. At 0.5 h, 2 h, 4 h, 8 h, and 24 h, 1.0 mL of the sample was removed and replaced with a new receiver solution. At 24 h, the skin was collected. The pig skin was treated, the epidermal and dermis layers were separated and extracted with a skin extract solution, and the residual drug amount, drug concentrations in the epidermal and dermis layers, and drug penetration concentration were detected by HPLC.

### Experimental parameters of IVPT:

| Item | Parameter |
|---|---|
| Receiver solution | pH 7.0, 50 mM sodium citrate + 10% HP-β-CD |
| Receiver cell volume | 8.0 mL |
| Sample amount | 60 mg |
| Receiving area | 3.14 cm² |
| Temperature | 32.0±1 °C |
| Rotor speed | 250 rpm |
| Sampling volume | 1.0 mL |
| Supplementary liquid volume | 1.0 mL |
| Sampling time point | 0.5, 2, 4, 8, 24 h |
| Residual drug and skin extract solution | Acetonitrile:water:1 M NaOH solution = 40:60:1 |

| Batch No. | Example 11.1 | Example 11.2 | Example 11.3 | Example 11.4 |
|---|---|---|---|---|
| Epidermis drug amount/µg | 31.5±8.6 | 18.7±5.9 | 7.1±2.4 | 20.6±4.5 |
| Dermis drug amount/µg | 154.5±21.4 | 75.5±17.7 | 47.0±4.7 | 51.2±12.2 |

Compared with the solution in the blank group, lactic acid showed a relatively good skin penetration-enhancing effect, and meanwhile, propylene glycol monocaprylate also showed a certain skin penetration-enhancing effect.

### Example 13

The active ingredient was dissolved in the formulation amount of dimethyl sulfoxide until it became a clear solution for later use. The formulation amount of hydroxypropyl cellulose was uniformly swelled in the formulation amount of diethylene glycol monoethyl ether. The formulation amounts of other non-aqueous solvents propylene glycol, ethanol, and the like were separately added, and the mixture was stirred uniformly. The dimethyl sulfoxide solvent and the diethylene glycol monoethyl ether phase were mixed. The specific components are as follows:

**Table 11**

| Component | Proportion (%) and Batch No. | |
|---|---|---|
| | Example 13.1 | Example 13.2 |
| Bisulfate of active ingredient | 1 | 0.5 |
| Dimethyl sulfoxide | 35 | 35 |
| Diethylene glycol monoethyl ether | 10 | 10 |
| Propylene glycol | 12.5 | 15.5 |
| Absolute ethanol | 10 | 10 |
| Water | 25 | 25 |
| Hydroxypropyl cellulose | / | 2 |
| Penetration Enhancer | Lactic acid | Laureth-4 |
| | 7 | 4 |
| pH(1 mol/L NaOH) | 3.5-4.0 | |

### Example 14

The penetrability of the API in the topical formulations was studied using freshly excised skin samples from Bama miniature pigs mounted in Franz diffusion cells. The fresh skin of Bama miniature pigs was depilated and then stored at -20 °C for later use. On the morning of the experiment, the skin was taken out and thawed, and after the transepidermal water loss value of the skin was detected to be less than 30, the skin was ready for use. The skin was cut to a size suitable for placing the pig skin samples between the donor and receiver compartments, and the pig skin samples were placed between the donor and receiver compartments of the Franz diffusion cell. About 15 mg of the sample was evenly applied to the pig skin. The opening of the Franz cell was 1 cm². 8.0 mL of a receiver solution was used. At 0.5 h, 2 h, 4 h, 8 h, and 24 h, 1.5 mL of the sample was removed and replaced with a new receiver solution. At 24 h, the skin was collected. The pig skin was treated, the epidermal and dermis layers were separated and extracted with a skin extract solution, and the residual drug amount, drug concentrations in the epidermal and dermis layers, and drug penetration concentration were detected by HPLC.

### Experimental parameters of IVPT:

| Item | Parameter |
|---|---|
| Receiver solution | pH 7.0, 50 mM sodium citrate + 10% HP-β-CD |
| Receiver cell volume | 8.0 mL |
| Sample amount | 15 mg |
| Receiving area | 1.0 cm² |
| Temperature | 32.0±1 °C |
| Rotor speed | 250 rpm |
| Sampling volume | 1.5 mL |
| Supplementary liquid volume | 1.5 mL |
| Sampling time point | 0.5, 2, 4, 8, 24 h |
| Residual drug and skin extract solution | Acetonitrile:water:1 M NaOH solution = 40:60:1 |

| Batch No. | Example 13.1 (see Example 13) | Example 13.2 (see Example 13) | Example 9.1 (see Example 9) |
|---|---|---|---|
| Epidermis drug amount/µg | 35.2±8.2 | 8.0±1.0 | 30.6±6.8 |
| Dermis drug amount/µg | 32.0±8.9 | 1.3±0.6 | 6.1±2.7 |
| Drug penetration amount/µg | 1.0±0.6 | 0.8±0.1 | 0.8±0.2 |

### Example 15

The active ingredient and butylated hydroxytoluene were dissolved in the formulation amount of dimethyl sulfoxide until it became a clear solution, and then the formulation amounts of lactic acid and other liquid excipients such as propylene glycol and glycerol were added. The mixture was stirred and mixed uniformly, and then the pH was adjusted to 3.5-4.5. The formulation amount of hydroxypropyl cellulose was added and uniformly swelled in the drug-containing solution. The specific components are shown in the table below:

**Table 12**

| Component | Proportion (%) and Batch No. | |
|---|---|---|
| | Example 15.1 | Example 15.2 |
| Active ingredient in free base form | 1 | 0.5 |
| Dimethyl sulfoxide | 35 | 35 |
| Diethylene glycol monoethyl ether | 10 | 10 |
| Lactic acid | 7 | 7 |
| Propylene glycol | 20 | 20 |
| PEG400 | 9 | 9 |
| Glycerol | 15.8 | 16.3 |
| Hydroxypropyl cellulose | 2 | 2 |
| Butylated hydroxytoluene | 0.2 | 0.2 |

### Example 16

The penetrability of the API in the topical formulations was studied using freshly excised skin samples from Bama miniature pigs mounted in Franz diffusion cells. The fresh skin of Bama miniature pigs was depilated and then stored at -20 °C for later use. On the morning of the experiment, the skin was taken out and thawed, and after the transepidermal water loss value of the skin was detected to be less than 30, the skin was ready for use. The skin was cut to a size suitable for placing the pig skin samples between the donor and receiver compartments, and the pig skin samples were placed between the donor and receiver compartments of the Franz diffusion cell. About 15 mg of the sample was evenly applied to the pig skin. The opening of the Franz cell was 1 cm². 8.0 mL of a receiver solution was used. At 0.5 h, 2 h, 4 h, 8 h, and 24 h, 1.5 mL of the sample was removed and replaced with a new receiver solution. At 24 h, the skin was collected. The pig skin was treated, the epidermal and dermis layers were separated and extracted with a skin extract solution, and the residual drug amount, drug concentrations in the epidermal and dermis layers, and drug penetration concentration were detected by HPLC.

### Experimental parameters of IVPT:

| Item | Parameter | |
|---|---|---|
| Receiver solution | pH 7.0, 50 mM sodium citrate + 10% HP-β-CD | |
| Receiver cell volume | 8.0 mL | |
| Sample amount | 15 mg | |
| Receiving area | 1.0 cm² | |
| Temperature | 32.0±1 °C | |
| Rotor speed | 250 rpm | |
| Sampling volume | 1.5 mL | |
| Supplementary liquid volume | 1.5 mL | |
| Sampling time point | 0.5, 2, 4, 8, 24 h | |
| Residual drug and skin extract solution | Acetonitrile:water:1 M NaOH solution = 40:60:1 | |

| Sample | Example 15.1 (see Example 15) | Example 15.2 (see Example 15) |
|---|---|---|
| Epidermis drug amount/µg | 27.6±12.0 | 10.4±7.5 |
| Dermis drug amount/µg | 11.8±10.1 | 7.8±0.5 |

## Claims

1. A pharmaceutical composition, comprising the following components: an active ingredient, dimethyl sulfoxide, and a penetration enhancer, wherein the active ingredient is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof; the penetration enhancer is one or more penetration enhancers selected from lactic acid, isopropyl myristate, and propylene glycol monocaprylate.

2. The pharmaceutical composition according to claim 1, wherein, based on the total mass of the pharmaceutical composition, the content of dimethyl sulfoxide is 20%-80%, and the content of the active ingredient is 0.3%-2.0%; the content of water in the pharmaceutical composition is less than 10%.

3. The pharmaceutical composition according to claim 1 or 2, wherein, based on the total mass of the pharmaceutical composition, the content of the active ingredient is 0.5%-2.0%, and the active ingredient is preferably present in a dissolved form in the pharmaceutical composition.

4. The pharmaceutical composition according to claim 1 or 2, wherein, based on the total mass of the pharmaceutical composition, the content of dimethyl sulfoxide is 25%-55%, preferably 30%-45%.

5. The pharmaceutical composition according to claim 1 or 2, wherein the pharmaceutical composition satisfies one or more of the following conditions:
(1) based on the total mass of the pharmaceutical composition, the content of the penetration enhancer is 1%-10%, preferably 5%-10%;
(2) based on the total mass of the pharmaceutical composition, the content of water is less than 9%, preferably less than 8%, and more preferably less than 7%.

6. The pharmaceutical composition according to claim 1 or 2, further comprising one or more of the following components:
(1) an organic solvent component other than dimethyl sulfoxide;
(2) a matrix;
(3) an antioxidant;
(4) a moisturizer;
(5) a preservative;
(6) a thickener; and
(7) a taste-masking agent.

7. The pharmaceutical composition according to claim 6, wherein the pharmaceutical composition satisfies one or more of the following conditions:
(1) the organic solvent component is one or more organic solvent components independently selected from benzyl alcohol, oleic acid, oleyl alcohol, diethylene glycol monoethyl ether, ethanol, and propylene glycol;
(2) the matrix is a water-soluble matrix, the water-soluble matrix is a polyethylene glycol polymer compound, and the polyethylene glycol polymer compound preferably has an average molecular weight of 100-6000;
(3) the antioxidant is one or more of butylated hydroxyanisole, butylated hydroxytoluene, propyl gallate, and tocopherol;
(4) the moisturizer is one or more of glycerol, propylene glycol, 1,3-butanediol, sorbitol, xylitol, hyaluronic acid, and trehalose;
(5) the preservative is one or more of methylparaben, ethylparaben, benzoic acid, sorbic acid, phenoxyethanol, chlorobutanol, phenylmercuric acetate, phenol, cresol, and benzalkonium chloride;
(6) the thickener is one or more of xanthan gum, hydroxyethyl cellulose, hydroxypropyl cellulose, methyl cellulose, sodium alginate, and poloxamer; and
(7) the taste-masking agent is one or more of strawberry essence, orange essence, and cyclodextrin.

8. The pharmaceutical composition according to claim 6, wherein the pharmaceutical composition satisfies one or more of the following conditions:
(1) based on the total mass of the pharmaceutical composition, the content of the organic solvent component is 10%-80%, preferably 10%-60%;
(2) based on the total mass of the pharmaceutical composition, the content of the matrix is 30%-80%, preferably 40%-50%, or the content of the matrix is 1%-20%, preferably 5%-15%;
(3) based on the total mass of the pharmaceutical composition, the content of the antioxidant is 0.05%-0.5%, preferably 0.05%-0.2%;
(4) based on the total mass of the pharmaceutical composition, the content of the moisturizer is 1%-50%, preferably 1%-25%;
(5) based on the total mass of the pharmaceutical composition, the content of the preservative is 0.05%-0.5%; and
(6) based on the total mass of the pharmaceutical composition, the content of the thickener is 0.1%-5%, preferably 0.5%-3%.

9. The pharmaceutical composition according to claim 1 or 2, wherein the pharmaceutical composition is any one of the following schemes:
scheme I: the pharmaceutical composition comprises an active ingredient, dimethyl sulfoxide, an organic solvent, a thickener, an antioxidant, and a penetration enhancer, wherein the organic solvent is one or more of benzyl alcohol, oleic acid, oleyl alcohol, diethylene glycol monoethyl ether, ethanol, and propylene glycol; the thickener is one or more of xanthan gum, hydroxyethyl cellulose, hydroxypropyl cellulose, methyl cellulose, and poloxamer; the antioxidant is one or more of butylated hydroxyanisole, butylated hydroxytoluene, propyl gallate, and tocopherol; the penetration enhancer is one or more penetration enhancers selected from lactic acid, isopropyl myristate, and propylene glycol monocaprylate;
scheme II: the pharmaceutical composition comprises an active ingredient, dimethyl sulfoxide, an organic solvent, a water-soluble matrix, and a penetration enhancer, wherein the organic solvent is one or more of benzyl alcohol, oleic acid, oleyl alcohol, diethylene glycol monoethyl ether, ethanol, and propylene glycol; the water-soluble matrix is a polyethylene glycol polymer compound; the penetration enhancer is one or more penetration enhancers selected from lactic acid, isopropyl myristate, and propylene glycol monocaprylate;
scheme III: the pharmaceutical composition comprises an active ingredient, dimethyl sulfoxide, a penetration enhancer, and an organic solvent, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol;
scheme IV: the pharmaceutical composition comprises an active ingredient, dimethyl sulfoxide, a penetration enhancer, an organic solvent, a thickener, and a water-soluble matrix, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol; the thickener is one or more of xanthan gum, hydroxyethyl cellulose, hydroxypropyl cellulose, methyl cellulose, and poloxamer; the water-soluble matrix is a polyethylene glycol polymer compound;
scheme V: the pharmaceutical composition comprises an active ingredient, dimethyl sulfoxide, a penetration enhancer, an organic solvent, a thickener, a water-soluble matrix, and an antioxidant, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol; the thickener is one or more thickeners selected from carbomer and siloxane substances; the water-soluble matrix is a polyethylene glycol polymer compound; the antioxidant is one or more of butylated hydroxyanisole, butylated hydroxytoluene, propyl gallate, and tocopherol;
scheme VI: the pharmaceutical composition comprises an active ingredient, dimethyl sulfoxide, an organic solvent, a penetration enhancer, a water-soluble matrix, a thickener, a moisturizer, and an antioxidant, wherein the organic solvent is one or more of benzyl alcohol, oleic acid, oleyl alcohol, diethylene glycol monoethyl ether, ethanol, and propylene glycol; the penetration enhancer is one or more penetration enhancers selected from lactic acid, isopropyl myristate, and propylene glycol monocaprylate; the water-soluble matrix is a polyethylene glycol polymer compound; the thickener is one or more of xanthan gum, hydroxyethyl cellulose, hydroxypropyl cellulose, methyl cellulose, sodium alginate, and poloxamer; the moisturizer is one or more of glycerol, propylene glycol, 1,3-butanediol, sorbitol, xylitol, hyaluronic acid, and trehalose; the antioxidant is one or more of butylated hydroxyanisole, butylated hydroxytoluene, propyl gallate, and tocopherol.

10. The pharmaceutical composition according to claim 9, wherein the pharmaceutical composition is any one of the following schemes:
scheme I: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, 10%-80% by mass of an organic solvent, 0.1%-5% by mass of a thickener, 0.05%-0.5% by mass of an antioxidant, and 1%-10% by mass of a penetration enhancer, wherein the organic solvent is one or more of benzyl alcohol, oleic acid, oleyl alcohol, diethylene glycol monoethyl ether, ethanol, and propylene glycol; the thickener is one or more of xanthan gum, hydroxyethyl cellulose, hydroxypropyl cellulose, methyl cellulose, and poloxamer; the antioxidant is one or more of butylated hydroxyanisole, butylated hydroxytoluene, propyl gallate, and tocopherol; the penetration enhancer is one or more penetration enhancers selected from lactic acid, isopropyl myristate, and propylene glycol monocaprylate;
scheme II: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, 10%-80% by mass of an organic solvent, 30%-80% by mass of a water-soluble matrix, and 1%-10% by mass of a penetration enhancer, wherein the organic solvent is one or more of benzyl alcohol, oleic acid, oleyl alcohol, diethylene glycol monoethyl ether, ethanol, and propylene glycol; the water-soluble matrix is a polyethylene glycol polymer compound; the penetration enhancer is one or more penetration enhancers selected from lactic acid, isopropyl myristate, and propylene glycol monocaprylate;
scheme III: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, and 1%-10% by mass of a penetration enhancer, wherein the penetration enhancer is one or more penetration enhancers selected from lactic acid, isopropyl myristate, and propylene glycol monocaprylate;
scheme IV: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, 1%-10% by mass of a penetration enhancer, and 10%-80% by mass of an organic solvent, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol;
scheme V: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, 1%-10% by mass of a penetration enhancer, 10%-80% by mass of an organic solvent, 0.1%-15% by mass of a thickener, and 1%-20% or 30%-80% by mass of a water-soluble matrix, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol; the thickener is one or more of xanthan gum, hydroxyethyl cellulose, hydroxypropyl cellulose, methyl cellulose, and poloxamer; the water-soluble matrix is a polyethylene glycol polymer compound;
scheme VI: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, 1%-10% by mass of a penetration enhancer, 10%-80% by mass of an organic solvent, 0.1%-15% by mass of a thickener, 1%-20% by mass of a water-soluble matrix, and 0.05%-0.5% by mass of an antioxidant, wherein the penetration enhancer is one or more penetration enhancers selected from lactic acid, isopropyl myristate, and propylene glycol monocaprylate; the organic solvent is one or more of benzyl alcohol, oleic acid, oleyl alcohol, diethylene glycol monoethyl ether, ethanol, and propylene glycol; the thickener is one or more thickeners selected from carbomer and siloxane substances; the water-soluble matrix is a polyethylene glycol polymer compound; the antioxidant is one or more of butylated hydroxyanisole, butylated hydroxytoluene, propyl gallate, and tocopherol;
scheme VII: the pharmaceutical composition comprises 0.3%-2.0% by mass of an active ingredient, which is (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide or a pharmaceutically acceptable salt thereof, 20%-80% by mass of dimethyl sulfoxide, 10%-80% by mass of an organic solvent, 1%-10% by mass of a penetration enhancer, 1%-20% by mass of a water-soluble matrix, 0.1%-5% by mass of a thickener, 0.05%-0.5% by mass of an antioxidant, and 1%-50% by mass of a moisturizer, wherein the organic solvent is one or more of benzyl alcohol, oleic acid, oleyl alcohol, diethylene glycol monoethyl ether, ethanol, and propylene glycol; the penetration enhancer is one or more penetration enhancers selected from lactic acid, isopropyl myristate, and propylene glycol monocaprylate; the water-soluble matrix is a polyethylene glycol polymer compound; the thickener is one or more of xanthan gum, hydroxyethyl cellulose, hydroxypropyl cellulose, methyl cellulose, sodium alginate, and poloxamer; the moisturizer is one or more of glycerol, propylene glycol, 1,3-butanediol, sorbitol, xylitol, hyaluronic acid, and trehalose; the antioxidant is one or more of butylated hydroxyanisole, butylated hydroxytoluene, propyl gallate, and tocopherol.

11. The pharmaceutical composition according to claim 10, wherein the pharmaceutical composition comprises components in any one of the following groups:
(1) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 40% by mass of dimethyl sulfoxide, 49.1% by mass of diethylene glycol monoethyl ether, 0.3% by mass of benzyl alcohol, 2% by mass of hydroxypropyl cellulose, 0.1% by mass of butylated hydroxytoluene, and 7.5% by mass of isopropyl myristate;
(2) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 40% by mass of dimethyl sulfoxide, 49.1% by mass of diethylene glycol monoethyl ether, 3.3% by mass of oleic acid, 1% by mass of hydroxypropyl cellulose, 0.1% by mass of butylated hydroxytoluene, and 5.5% by mass of isopropyl myristate;
(3) 0.5% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 5% by mass of diethylene glycol monoethyl ether, 5% by mass of propylene glycol, 2% by mass of benzyl alcohol, 7% by mass of lactic acid, 10.5% by mass of polyethylene glycol 400, and 35% by mass of polyethylene glycol 4000;
(4) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 4% by mass of propylene glycol, 7% by mass of lactic acid, 4% by mass of polyethylene glycol 400, and 40% by mass of polyethylene glycol 4000;
(5) 1.5% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 4% by mass of propylene glycol, 7% by mass of lactic acid, 9% by mass of polyethylene glycol 400, and 35% by mass of polyethylene glycol 4000;
(6) 1.855% by mass of bisulfate of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 2.145% by mass of propylene glycol, 7% by mass of lactic acid, 9% by mass of polyethylene glycol 400, and 35% by mass of polyethylene glycol 4000;
(7) 1% by mass of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 4% by mass of propylene glycol, 7% by mass of lactic acid, 9% by mass of polyethylene glycol 400, and 35% by mass of polyethylene glycol 4000;
(8) 1% by mass of bisulfate of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 10% by mass of diethylene glycol monoethyl ether, 12.5% by mass of propylene glycol, 10% by mass of ethanol, and 7% by mass of lactic acid;
(9) 0.37% by mass of bisulfate of (3aR,5s,6aS)-N-(3-methoxy-1,2,4-thiadiazol-5-yl)-5-(methyl(7H-pyrrolo[2,3-d]pyrimidin-4-yl)amino)hexahydrocyclopenta[c]pyrrole-2(1H)-carboxamide, 35% by mass of dimethyl sulfoxide, 0.05% by mass of butylated hydroxytoluene, 2% by mass of benzyl alcohol, 9.63% by mass of propylene glycol, 7% by mass of lactic acid, 12.5% by mass of polyethylene glycol 400, 2.3% by mass of carbomer, 7% by mass of cyclic dimethylsiloxane, 1% by mass of poly(dimethylsiloxane), and 2% by mass of ST-Elastomer 10.

12. The pharmaceutical composition according to any one of claims 1-11, wherein the pharmaceutically acceptable salt is one or more of hydrochloride, maleate, hydrobromide, p-toluenesulfonate, mesylate, sulfate, bisulfate, and ethanesulfonate, preferably bisulfate.

13. The pharmaceutical composition according to any one of claims 1-12, wherein the pharmaceutical composition is a pharmaceutical composition for topical use; preferably, the pharmaceutical composition is a gel, an emulsion, or an ointment, and more preferably, the pharmaceutical composition is a gel.

14. A method for preparing the pharmaceutical composition according to any one of claims 1-13, comprising: mixing the components, wherein preferably, the method comprises: mixing the active ingredient and dimethyl sulfoxide with the penetration enhancer, and further mixing with optionally at least one of the organic solvent, the matrix, and the antioxidant.

15. Use of the pharmaceutical composition according to any one of claims 1-13 in the manufacture of a medicament for treating or preventing immune system disorders or diseases, wherein preferably, the immune system disorders or diseases are selected from organ transplant rejection, ankylosing spondylitis, active non-radiographic axial spondyloarthritis, rheumatoid arthritis, psoriatic arthritis, ulcerative colitis, psoriasis, vitiligo, alopecia areata, atopic dermatitis, Crohn's disease, and lymphoma; more preferably, the immune system disorders or diseases are selected from vitiligo.
